# EUROPEAN PATENT SPECIFICATION

(11) **EP 4 117 462 B1**
(45) Date of publication and mention of the grant of the patent: **16.09.2026**
(21) Application number: 21716844.2
(22) Date of filing: 01.04.2021
(51) Int. Cl.: A24B 15/167, A24F 40/05, A24F 40/10, A61M 11/00, A61M 15/00, B05B 17/06, B05B 17/00

(54) **CELL LYSIS SYSTEMS AND METHODS**
ZELLSYSTEME UND METHODEN ZUR LYSE VON ZELLEN
SYSTÈMES ET MÉTHODES DE LYSE CELLULAIRE

(30) Priority: 06.04.2020 EP 20168245; 01.06.2020 US 202016889667; 08.10.2020 US 202017065992; 09.11.2020 US 202063111592 P; 15.12.2020 US 202017122025
(43) Date of publication of application: 18.01.2023
(60) Divisional application: 22207045.0 / 4 159 053
(73) Proprietor: Shaheen Innovations Holding Limited, Abu Dhabi (AE)
(72) Inventor: MACHOVEC, Jeff, Abu Dhabi Global Market Square Al Maryah Island, Abu Dhabi (AE); ALSHAIBA SALEH GHANNAM ALMAZROUEI, Mohammed, Abu Dhabi Global Market Square Al Maryah Island, Abu Dhabi (AE); LAMOUREUX, Clement, Abu Dhabi Global Market Square Al Maryah Island, Abu Dhabi (AE)
(74) Representative: Forresters IP LLP
(86) International application number: PCT/GB2021/050822
(87) International publication number: WO 2021/205151

(56) References cited:
- EP-A2- 0 353 365
- EP-A2- 1 179 585
- WO-A1-2019/130107
- US-A- 6 100 084
- US-A1- 2004 042 936
- US-A1- 2018 207 551
- US-B2- 6 878 540

## Description

### Field

The present invention relates to cell lysis systems and methods. The present invention more particularly relates to cell lysis systems and methods which use ultrasonic waves to lyse cells.

### Background

Polymerase Chain Reaction (PCR) is a process that uses the two matching strands in DNA to amplify a targeted DNA sequence from a small number of samples to billions of copies for analysis.

An initial step of the PCR process involves cell lysis to break or rupture the lipid bilayer of cells in a sample in order to provide a gateway through which a cell's components, including DNA and/or RNA, may be extracted. Cell lysis is typically performed either chemically or electromechanically, or a combination of both.

The cell lysis process extracts components from the cells in a liquid solution. The solution is then filtered to separate the nucleic acids (DNA/RNA) from other cell components. The extracted DNA/RNA can then be amplified and analysed in the remaining steps of the PCR process.

A conventional PCR apparatus performs cell lysis on a sample when the sample is input into the PCR apparatus. The components performing the cell lysis process are typically integrated within the PCR apparatus. The problem with a conventional PCR apparatus of this kind is that the apparatus is typically expensive and cumbersome. Moreover, the integrated components which perform the cell lysis are typically restricted for use only within the same PCR apparatus.

Standalone cell lysis devices have been proposed previously but these standalone devices can suffer from reduced efficiency and performance compared with the cell lysis functionality of a complete PCR apparatus.

Thus, a need exists in the art for improved cell lysis systems and methods which seek to address at least some of the problems described herein.

EP 1 179 585 A2 discloses a cartridge for the processing of a fluid sample.

### Summary

The present invention provides a system for the lysis of cells as claimed in claim 1 and a method as claimed in claim 10.

### Brief description of the drawings

So that the present invention may be more readily understood, embodiments of the present invention will now be described, by way of example, with reference to the accompanying drawings, in which:
Figure 1 is a diagrammatic perspective view of a system of some arrangements;
Figure 2 is a diagrammatic perspective view of a cell lysis device of some arrangements;
Figure 3 is a cross-sectional view of the cell lysis device of Figure 2;
Figure 4 is a diagrammatic perspective view of a driver apparatus of some arrangements;
Figure 5 is a cross-sectional view of the driver apparatus of Figure 4;
Figure 6 is a diagram showing a piezoelectric transducer modelled as an RLC circuit;
Figure 7 is a graph showing the change in impedance with increase in frequency in an RLC circuit;
Figure 8 is a graph showing how a piezoelectric transducer acts as a capacitor or an inductor; and
Figure 9 is a table showing the timings of operating modes of a system of some arrangements.

### Detailed description

Aspects of the present disclosure are best understood from the following detailed description when read with the accompanying figures. It is noted that, in accordance with the standard practice in the industry, various features are not drawn to scale. In fact, the dimensions of the various features may be arbitrarily increased or reduced for clarity of discussion.

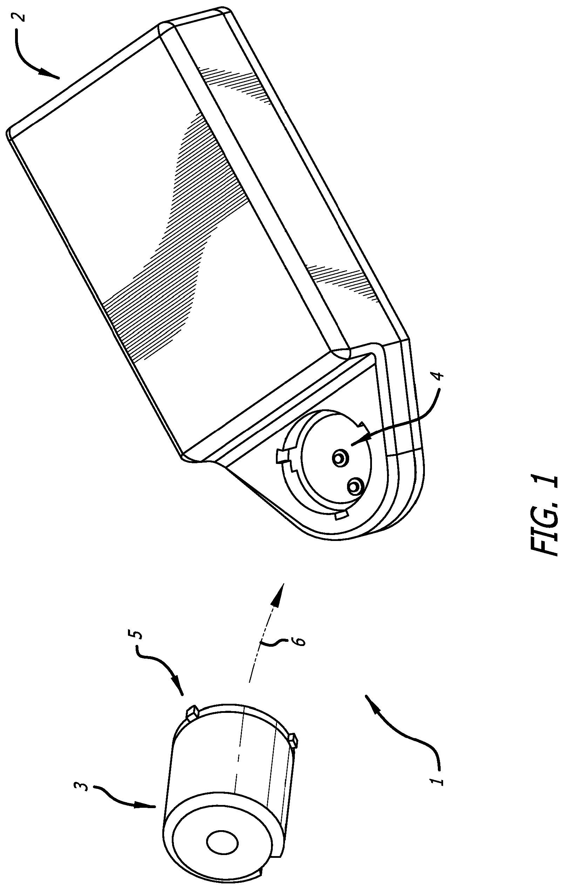

Referring initially to Figure 1 of the accompanying drawings, a cell lysis system 1 of some arrangements comprises a driver apparatus 2 and a cell lysis device 3. The driver apparatus 2 comprises a first interference fit attachment 4 which releasably attaches to a second interference fit attachment 5 provided on the cell lysis device 3. The interference fit attachments 4, 5 allow the cell lysis device 3 to be releasably attached to the driver apparatus 2 when the cell lysis device 3 is placed onto the driver apparatus 2 as indicated generally by arrow 6 in Figure 1.

The components of the cell lysis system 1 are described below, starting with the cell lysis device 3.

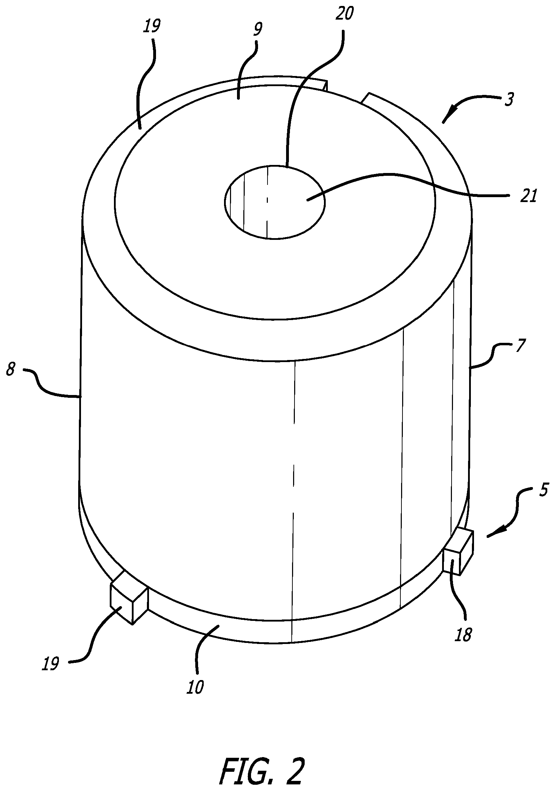

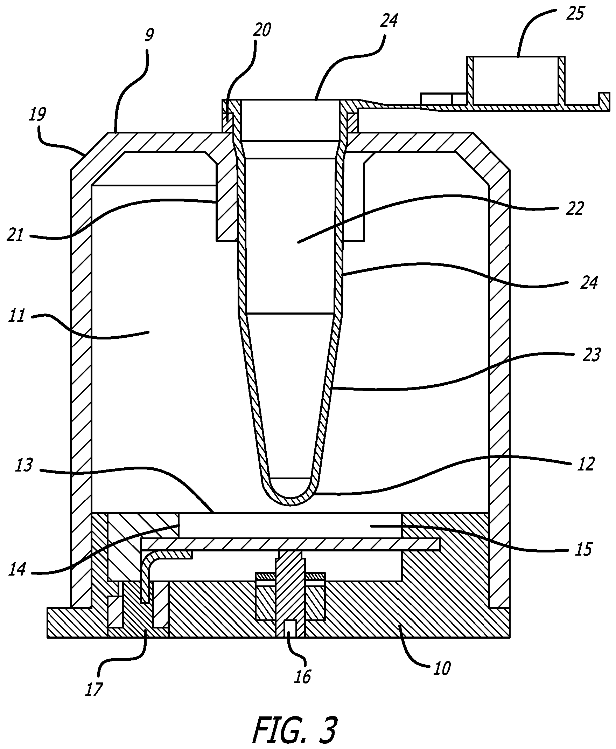

Referring to Figures 2 and 3 of the accompanying drawings, the cell lysis device 3 of some arrangements comprises a housing 7. In this arrangement, the housing 7 is generally cylindrical with a side wall 8, a generally circular cover 9 at one end and a generally circular base 10 at the other end. In this arrangement, the cell lysis device 3 is a disposable, single-use capsule.

A sonication chamber 11 is provided within the housing 7. The sonication chamber 11 is at least partly filled with an ultrasonic wave transfer medium (not shown). In some arrangements, the sonication chamber 11 is pre-filled with the ultrasonic wave transfer medium. In other arrangements, the sonication chamber 11 is filled with an ultrasonic wave transfer medium when the cell lysis device is being prepared for use.

In some arrangements, the ultrasonic wave transfer medium is a liquid which has a higher acoustic impedance than water. In some arrangements, the ultrasonic wave transfer medium is vegetable glycerine since vegetable glycerine has a higher acoustic impedance than water.

The cell lysis device 3 comprises an ultrasonic transducer 12. An upper surface 13 of the ultrasonic transducer 12 faces towards the sonication chamber 11 so that ultrasonic waves generated by the ultrasonic transducer 12 are directed towards the sonication chamber 11.

In some arrangements, the ultrasonic transducer 12 is a circular disc shape. In this arrangement, the ultrasonic transducer 12 has a diameter of approximately 16mm and a thickness of approximately 0.7mm. In this arrangement, the ultrasonic transducer 12 is polarized to generate vibrations in the thickness mode.

In this arrangement, the ultrasonic transducer 12 is carried by a transducer holder 14 in the form of a ring which at least partly surrounds the ultrasonic transducer 12. In this arrangement, the transducer holder 14 is of silicone rubber. Apart from holding the ultrasonic transducer 12 in place, the transducer holder 14 also ensures minimal damping of the vibration of the ultrasonic transducer 12. In addition, the transducer holder 14 minimises the risk of the liquid ultrasonic wave transfer medium from leaking out from the base 10 of the cell lysis device 3.

In some arrangements, the ultrasonic transducer 12 is at least partly of a compound comprising lead, zirconium and titanium. The compound of the ultrasonic transducer 12 is selected to provide the ultrasonic transducer 12 with the properties for it to oscillate at a frequency of 2.8MHz to 3.2MHz. This frequency range is the preferred frequency range for the ultrasonic transducer 12 to produce ultrasonic waves which lyse or rupture cells.

In some arrangements, the ultrasonic transducer 12 comprises a first electrode on the upper side 13 and a second electrode on a lower side 15 which is on the opposing side of the ultrasonic transducer 12. In some arrangements, the first electrode and the second electrode comprise silver, for instance in the form of silver stamp paint. In some arrangements, the capacitance between the first electrode and the second electrode is 800pF to 1300pF.

In some arrangements, the first electrode on the upper side 13 of the ultrasonic transducer 12 is at least partly covered with a glass coating. The glass coating minimizes or prevents possible contamination of the ultrasonic wave transfer medium by the material of the first electrode. The glass coating also minimizes or prevents erosion of the silver of the first electrode, for instance due to cavitation bubble collapse caused by ultrasonic waves travelling through the ultrasonic wave transfer medium when the cell lysis device 3 is in use.

The first and second electrodes of the ultrasonic transducer 12 are connected electrically to respective first and second electrical terminals 16, 17 which are provided at the lower surface of the base 10 of the cell lysis device 3.

A plurality of projections 18, 19 (only two of which are visible in Figure 2) extend outwardly from the side of the base 10 of the cell lysis device 3. The projections 18, 19 are part of the second interference fit attachment 5 of the cell lysis device 3. In other arrangement, the second interference fit attachment 5 comprises only one projection and in further arrangements, the second interference fit attachment 5 comprises more than 2 projections.

The cover 9 is provided at the opposite end of the cell lysis device 3 to the base 10. The cover 9 provides a generally planar circular surface and is formed integrally with the side wall 8 of the housing 7. In this arrangement, a bevelled edge 19 is provided around the circumference of the cover 9 at the intersection between the cover 9 and the side wall 8.

An opening 20 is provided in the cover 9. In this arrangement, the opening 20 is a generally circular aperture which is provided at the centre of the cover 9. In other arrangements, the opening 20 may be a different shape and may be provided in a different portion of the cover 9.

In this arrangement, a cylindrical collar 21 is aligned with the opening 20 and extends from the opening 20 into the sonication chamber 11. The opening 20 and the collar 21 are configured to receive a sample container 22 such that a part of the sample container 22 projects into the ultrasonic wave transfer medium within the sonication chamber 11.

In some arrangement, the sample container 22 is a microcentrifuge tube. In some arrangement, the sample container 22 is an Eppendorf Tube^{®}. In some arrangements, the sample container 22 is a microcentrifuge tube or Eppendorf Tube^{®} which holds a liquid volume of 0.5ml, 1.5ml or 2ml.

The sample container 22 comprises a conical first portion 23 which is joined to a cylindrical body portion 24. A distal end of the body portion 24 is provided with a sample container aperture 24 through which a sample may be introduced into the sample container 22. A cap 25 is moveably mounted to the body portion 24, with the cap 25 being configured to seal the aperture 24 to retain a sample within the sample container 22.

The sample container 22 closes and seals the opening 20 when the sample container 22 is inserted into the opening 20. The housing 7 of the cell lysis device 3 is thus sealed to retain the ultrasonic wave transfer medium within the housing 7. In some arrangements, the sonication chamber 11 is initially empty and the sonication chamber 11 is filled with the ultrasonic wave transfer medium shortly before the sample container 22 is inserted into the opening 20.

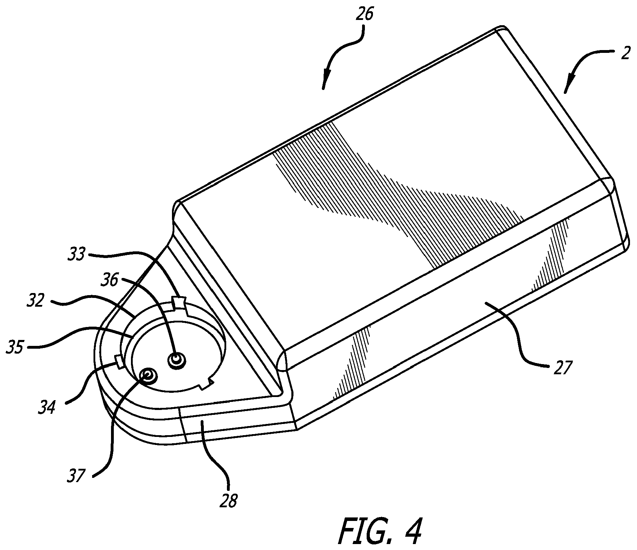

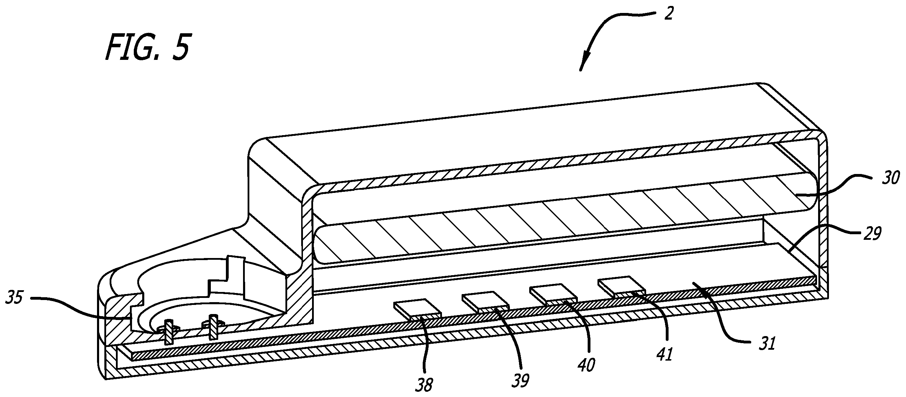

Referring now to Figures 4 and 5 of the accompanying drawings, the driver apparatus 2 comprises a housing 26 which houses electrical components of the driver apparatus 2. In this arrangement, the driver apparatus 2 is a portable, stand-alone apparatus.

The housing 26 comprises a main housing 27 and a base protrusion 28. The main housing 27 comprises an internal chamber 29 which receives a battery 30 and at least part of a printed circuit board (PCB) 31. The PCB 31 carries the electronic components which provide the driver functionality of the driver apparatus 2.

In this arrangement, the battery 30 is rechargeable. In some arrangements, the battery 30 is a lithium polymer (LiPo) battery. In some arrangements, the capacity of the battery 30 provides sufficient power to enable the driver apparatus 2 to operate for at least 24 hours. In this arrangement, the driver apparatus 2 comprises a charging connection (not shown) which is configured to receive power from an external power source to charge the battery 30.

In some arrangements, the battery 30 is omitted and the driver apparatus 2 is instead provided with a power input connection to receive power from an external power source. In some arrangements, the external power source is a power adapter which converts a mains voltage to an appropriate voltage (e.g. 5-12V) to power the driver apparatus 2.

The base protrusion 28 is, in this embodiment, of reduced thickness compared with the housing 26. The base protrusion 28 is provided with a recess 32 which is configured to receive the base 10 of the cell lysis device 3. In this arrangement, the recess 32 is generally circular. The recess 32 is provided with indentations 33, 34 which are positioned above a generally circular channel 35 to form the first interference fit attachment 4 of the driver apparatus 2. The indentations 33, 34 are positioned to align with the projections 18, 19 on the cell lysis device 3.

In other embodiments, there may be a different number of indentations which match a different number of projections on the cell lysis device 3.

Two driver output terminals 36, 37 are provided at the base of the recess 32. In this arrangement, one of the driver output terminals 36 is provided centrally within the recess 32 and the other driver output terminal 37 is provided adjacent the side of the recess 32. In this arrangement, each of the driver output terminals 36, 37 is a spring contact probe which protrudes upwardly from the base of the recess 32. The driver output terminals 36, 37 are positioned to engage and form an electrical connection with the first and second electrical terminals 16, 17 which are provided at the lower surface of the base 10 of the cell lysis device 3 when the cell lysis device 3 is attached to the driver apparatus 2. In some arrangements, the driver output terminals 36, 37 and/or the electrical terminals 16, 17 are of a brass material which is plated with a first 3µm thick layer of nickel and a second 0.05µm thick layer of gold.

In some arrangements, the driver output terminals 36, 37 extend through the base of the recess 32 to the PCB 31 where the driver output terminals 36, 37 are soldered to form an electrical connection with the PCB 31 and the electronic components provided on the PCB 31.

In this arrangement, the cell lysis device 3 is releasably attached to the driver apparatus 2 by placing the base 10 of the cell lysis device 3 into the recess 32 with the protrusions 18, 19 passing through the indentations 33, 34 until the protrusions 18, 19 are aligned with the channel 35. The cell lysis device 3 is then rotated with the protrusions 18, 19 within the channel 35. The protrusions 18, 19 are then retained within the channel 35 to provide an interference fit attachment which releasably attaches the cell lysis device 3 to the driver apparatus 2.

The interference fit attachments 4, 5 enable a user to releasably attach the cell lysis device 3 to the driver apparatus 2 by pushing and turning the cell lysis device 3 to lock the cell lysis device 3 to the driver apparatus. This process is then performed in reverse to remove the cell lysis device 3 from the driver apparatus 2 after the lysing process has finished.

As the cell lysis device 3 is pushed into the recess 32, the driver output terminals 36, 37 deform resiliently and align with the electrical terminals 16, 17 on the cell lysis device 3. The resiliently deformed driver output terminals 36, 37 press against the electrical terminals 16, 17 to form an electrical connection. When the cell lysis device 3 is releasably attached to the driver apparatus 2 in this way, the cell lysis device 3 is able to be driven by the driver apparatus 2 to lyse cells within the cell lysis device 3.

An AC driver 38 is provided on the PCB 31. The AC driver 38 generates an AC drive signal at a predetermined frequency and outputs the AC drive signal at the driver output terminals 36, 37 to drive the ultrasonic transducer 12 within the cell lysis device 3. **In** some arrangements, the AC driver 38 comprises a H-bridge circuit. **In** some arrangements, the H-bridge circuit comprises four MOSFETs which are connected to convert a direct current into an alternating current at high frequency (e.g. a frequency in the range 2.8MHz to 3.2MHz).

An active power monitoring arrangement 39 is provided on the PCB 31. The active power monitoring arrangement 39 monitors the active power used by the ultrasonic transducer 12 when the ultrasonic transducer 12 is driven by the AC drive signal. The active power monitoring arrangement 39 provides a monitoring signal which is indicative of an active power used by the ultrasonic transducer 12. **In** some arrangements, the active power monitoring arrangement 39 comprises a current sensing arrangement which senses a drive current of the AC drive signal driving the ultrasonic transducer 12 and provides a monitoring signal which is indicative of the sensed drive current.

The PCB 31 is provided with a processor 40 which controls the AC driver 38 and receives the monitoring signal from the active power monitoring arrangement 39.

The PCB 31 is also provided with a memory 41 storing executable instructions for execution by the processor 40.

In some arrangements, the driver apparatus 2 comprises a frequency controller which is configured to control the frequency at which the ultrasonic transducer 12 operates. The frequency controller is implemented in the executable code stored in the memory 41 which, when executed by the processor 40, cause the processor 40 to perform at least one function of the frequency controller.

The memory 41 stores executable instructions which, when executed by the processor, cause the processor to control the ultrasonic transducer 12 to oscillate at a plurality of frequencies within a predetermined sweep frequency range and to select a drive frequency for the ultrasonic transducer 12 which is between a first predetermined frequency and a second predetermined frequency for lysing cells within the sample container 22.

In some arrangements, the frequency will be determined by the type of cells that are being lysed as some cells may require different frequencies due to their physical characteristics (size, shape, presence of cell wall, etc.).

There is an optimum frequency or frequency range for lysing cells. The optimum frequency or frequency range will depend on at least the following four parameters:

### 1. Transducer Manufacturing Processes

In some arrangements, the ultrasonic transducer 12 comprises a piezoelectric ceramic. The piezoelectric ceramic is manufactured by mixing compounds to make a ceramic dough and this mixing process may not be consistent throughout production. This inconsistency can give rise to a range of different resonant frequencies of the cured piezoelectric ceramic.

If the resonant frequency of the piezoelectric ceramic does not correspond to the required frequency of operation, the process of lysing cells is not optimal. Even a slight offset in the resonant frequency of the piezoelectric ceramic is enough to impact the lysing process, meaning that the system will not function optimally.

### 2. Load on transducer

During operation, any changes in the load on the ultrasonic transducer 12 will inhibit the overall displacement of the oscillation of the ultrasonic transducer 12. To achieve optimal displacement of the oscillation of the ultrasonic transducer 12, the drive frequency must be adjusted to provide adequate power for maximum displacement.

The types of loads that can affect the efficiency of the ultrasonic transducer 12 can include the amount of liquid on the transducer (i.e. the amount of liquid within the sonication chamber 11).

### 3. Temperature

Ultrasonic oscillations of the ultrasonic transducer 12 are partially damped by its assembly in the driver apparatus 2. This dampening of the oscillations can cause a rise in local temperatures on and around the ultrasonic transducer 12.

An increase in temperature affects the oscillation of the ultrasonic transducer 12 due to changes in the molecular behaviour of the ultrasonic transducer 12. An increase in the temperature means more energy to the molecules of the ceramic, which temporarily affects its crystalline structure. Although the effect is reversed as the temperature reduces, a modulation in supplied frequency is required to maintain optimal oscillation.

An increase in temperature also reduces the viscosity of the solution within the sonication chamber 11, which may require an alteration to the drive frequency to optimise lysis of cells within the sonication chamber 11.

### 4. Distance to Power Source

The oscillation frequency of the ultrasonic transducer 12 can change depending on the wire-lengths between the ultrasonic transducer 12 and the AC driver 38. The frequency of the electronic circuit is inversely proportional to the distance between the ultrasonic transducer 49 and the controller 23.

Although the distance parameter is primarily fixed in this arrangement, it can vary during the manufacturing process of the system 1. Therefore, it is desirable to modify the drive frequency of the ultrasonic transducer 12 to compensate for the variations and optimise the efficiency of the system.

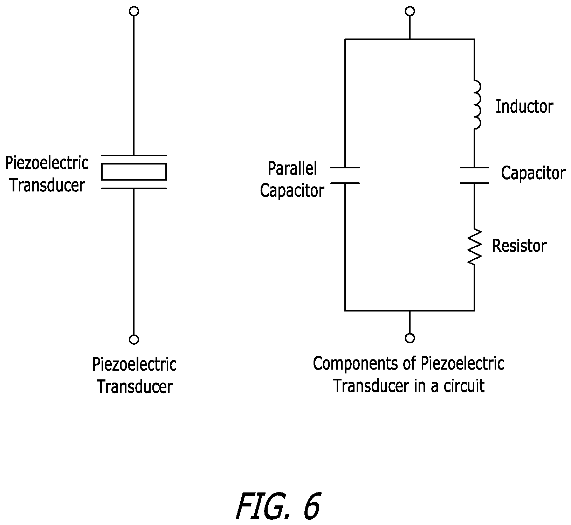

A piezoelectric transducer can be modelled as an RLC circuit in an electronic circuit, as shown in Figure 6. The four parameters described above may be modelled as alterations to the overall inductance, capacitance, and/or resistance of the RLC circuit, changing the resonance frequency range supplied to the transducer. As the frequency of the circuit increases to around the resonance point of the transducer, the log Impedance of the overall circuit dips to a minimum and then rises to a maximum before settling to a median range.

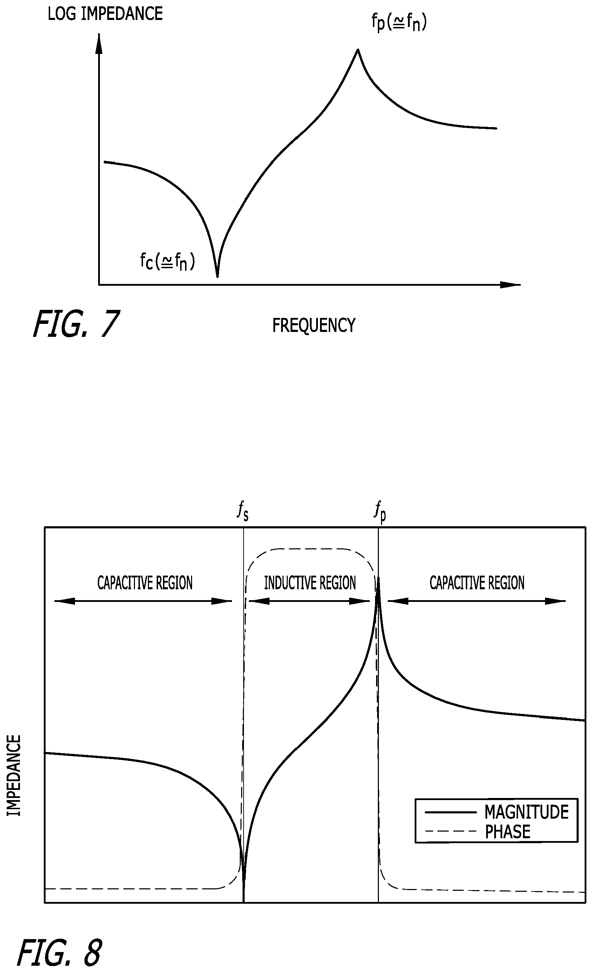

Figure 7 shows a graph explaining the change in overall impedance with increase in frequency in an RLC circuit. Figure 8 shows how a piezoelectric transducer acts as a capacitor in a first capacitive region at frequencies below a first predetermined frequency fₛ and in a second capacitive region at frequencies above a second predetermined frequency fₚ. The piezoelectric transducer acts as an inductor in an inductive region at frequencies between the first and second predetermined frequencies fₛ, fₚ. In order to maintain optimal oscillation of the transducer and hence maximum efficiency, the current flowing through the transducer must be maintained at a frequency within the inductive region.

The driver apparatus 2 of some arrangements is configured to maintain the frequency of oscillation of the piezoelectric transducer 12 within the inductive region, in order to maximise the efficiency of the lysis of cells.

The driver apparatus 2 is configured to perform a sweep operation in which the frequency controller drives the transducer at frequencies which track progressively across a predetermined sweep frequency range. In other words, the driver apparatus 2 drives the transducer at a plurality of different frequencies across the predetermined sweep frequency range. For instance at frequencies which increment by a predetermined frequency from one end of the sweep frequency range to the other end of the sweep frequency range.

As will be described in more detail below, the driver apparatus 2 of some arrangements determines the active power being used by the ultrasonic transducer 12 by monitoring the current flowing through the transducer 12.

Ultrasonic (piezoelectric) transducer mechanical deformation is linked to the AC Voltage amplitude that is applied to it, and in order to guarantee optimal functioning and delivery of the system, the maximum deformation must be supplied to the ultrasonic transducer all the time. By Pulse Width Modulation (PWM) of the AC voltage applied to the ultrasonic transducer, the mechanical amplitude of the vibration remains the same. In some arrangements, the system actively adjusts the duty cycle of the AC voltage waveform to maximise deformation of the ultrasonic transducer in order to guarantee optimal functioning and delivery of the system.

One approach involves modifying the AC voltage applied to the ultrasonic transducer via the use of a Digital to Analog Converter (DAC). The energy transmitted to the ultrasonic transducer would be reduced but so would the mechanical deformation which as a result does not produce maximum deformation. The RMS voltage applied to the ultrasonic transducer would be the same with effective Duty Cycle modulation as with Voltage modulation, but the active power transferred to the ultrasonic transducer would degrade. Indeed, given the formula below:
Active Power displayed to the ultrasonic transducer being: $Pa = \frac{2 \sqrt{2}}{π} Irms ∗ Vrms ∗ cosφ ,$ Where
*φ is* the shift in phase between current and voltage
Iᵣₘₛ is the root mean square Current
Vᵣₘₛ is the root mean square Voltage.

When considering the first harmonic, Irms is a function of the real voltage amplitude applied to the ultrasonic transducer, as the pulse width modulation alters the duration of voltage supplied to the ultrasonic transducer, controlling Irms.

**In** this arrangement, the memory 41 stores instructions which, when executed by the processor 40, cause the processor 40 to:
A. control the AC driver 38 to output an AC drive signal to the ultrasonic transducer 12 at a predetermined sweep frequency;
B. calculate the active power being used by the ultrasonic transducer 12 based on the monitoring signal;
C. control the AC driver 38 to modulate the AC drive signal to maximise the active power being used by the ultrasonic transducer 12;
D. store a record in the memory 41 of the maximum active power used by the ultrasonic transducer 12 and the sweep frequency of the AC drive signal;
E. repeat steps A-D for a predetermined number of iterations with the sweep frequency incrementing with each iteration such that, after the predetermined number of iterations has occurred, the sweep frequency has been incremented from a start sweep frequency to an end sweep frequency;
F. identify from the records stored in the memory 41 the optimum frequency for the AC drive signal which is the sweep frequency of the AC drive signal at which a maximum active power is used by the ultrasonic transducer 12; and
G. control the AC driver 38 to output an AC drive signal to the ultrasonic transducer 12 at the optimum frequency.

In some arrangements, the start sweep frequency is 2800kHz and the end sweep frequency is 3200kHz. In other arrangements, the start sweep frequency and the end sweep frequency are lower and upper frequencies of a frequency range within the range of 2800kHz to 3200kHz.

In some arrangements, the processor 40 controls the AC driver 38 to output an AC drive signal to the ultrasonic transducer 12 at frequency which is shifted by between 1-10% of the optimum frequency. In these arrangements, the frequency shift is used to prolong the life of the ultrasonic transducer 12 by minimising potential damage caused to the ultrasonic transducer 12 when the ultrasonic transducer 12 is driven continuously at the optimum drive frequency which produces maximum displacement.

In some arrangements, the AC driver 38 modulates the AC drive signal by pulse width modulation to maximise the active power being used by the ultrasonic transducer 12.

In some arrangements, the processor 40 controls the AC driver 38 to alternately output an AC drive signal to the ultrasonic transducer 12 at the optimum frequency for a first predetermined length of time and to not output an AC drive signal to the ultrasonic transducer 12 for a second predetermined length of time. This alternate activation and deactivation of the ultrasonic transducer 12 has been found to optimise the process of lysing cells in a sample within the cell lysis device 3.

In some embodiments, in order to ensure optimal operation of the ultrasonic transducer 12, the driver apparatus 2 operates in a recursive mode. When the driver apparatus 2 operates in the recursive mode, the driver apparatus 2 runs the sweep of frequencies in steps A-D periodically during the operation of the system.

In some arrangements, the driver apparatus 2 activates automatically to start the lysing process when the cell lysis device 3 is attached to the driver apparatus 2. In some arrangements, the driver apparatus 2 stops the lysing process automatically after a predetermined length of time. Once the lysing process has finished, the cell lysis device 3 is removed from the driver apparatus 3.

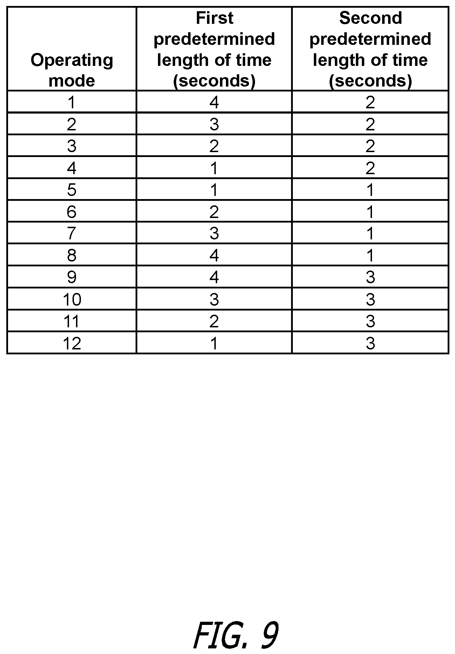

In some arrangements, the processor 40 controls the AC driver 38 to alternately output the AC drive signal and to not output the AC drive signal according to an operating mode. The timings of twelve operating modes of some arrangements are shown in the table in Figure 9 of the accompanying drawings.

In some arrangements, the driver apparatus 2 activates automatically when the cell lysis device 3 is attached to the driver apparatus 2. In other arrangements, the driver apparatus 2 is provided with a switch or other control device to enable a user to activate and deactivate the driver apparatus 2.

Once the system has been activated and has perfomed the lysing process for a predetermined duration, the cell lysis device 3 is separated from the driver apparatus 2. The liquid within the cell lysis device 3, which now contains lysed cells, is removed for use in another process, such as a PCR process. The cell lysis device 3 may then be discarded.

While the arrangements described above comprise one recess 32 and one set of driver output terminals 36, 37, other arrangements comprise a plurality of recesses and a plurality of sets of output terminals. In these other arrangments, the driver apparatus 2 can be used simultanously with a plurality of cell lysis devices. In these arrangements, the driver apparatus 2 controls each of the plurality of cell lysis devices to perform cell lysis individually.

The foregoing outlines features of several examples or embodiments so that those of ordinary skill in the art may better understand various aspects of the present disclosure. Those of ordinary skill in the art should appreciate that they may readily use the present disclosure as a basis for designing or modifying other processes and structures for carrying out the same purposes and/or achieving the same advantages of various examples or embodiments introduced herein. Those of ordinary skill in the art should also realise that such equivalent constructions do not depart from the spirit and scope of the present disclosure, and that they may make various changes, substitutions, and alterations herein without departing from the spirit and scope of the present disclosure.

Although the subject matter has been described in language specific to structural features or methodological acts, it is to be understood that the subject matter of the appended claims is not necessarily limited to the specific features or acts described above. Rather, the specific features and acts described above are disclosed as example forms of implementing at least some of the claims.

Various operations of examples or embodiments are provided herein. The order in which some or all of the operations are described should not be construed to imply that these operations are necessarily order dependent. Alternative ordering will be appreciated having the benefit of this description. Further, it will be understood that not all operations are necessarily present in each embodiment provided herein. Also, it will be understood that not all operations are necessary in some examples or embodiments.

Moreover, "exemplary" is used herein to mean serving as an example, instance, illustration, etc., and not necessarily as advantageous. As used in this application, "or" is intended to mean an inclusive "or" rather than an exclusive "or". **In** addition, "a" and "an" as used in this application and the appended claims are generally be construed to mean "one or more" unless specified otherwise or clear from context to be directed to a singular form. Also, at least one of A and B and/or the like generally means A or B or both A and B. Furthermore, to the extent that "includes", "having", "has", "with", or variants thereof are used, such terms are intended to be inclusive in a manner similar to the term "comprising". Also, unless specified otherwise, "first," "second," or the like are not intended to imply a temporal aspect, a spatial aspect, an ordering, etc. Rather, such terms are merely used as identifiers, names, etc. for features, elements, items, etc. For example, a first element and a second element generally correspond to element A and element B or two different or two identical elements or the same element.

Also, although the disclosure has been shown and described with respect to one or more implementations, equivalent alterations and modifications will occur to others of ordinary skill in the art based upon a reading and understanding of this specification and the annexed drawings. The disclosure comprises all such modifications and alterations and is limited only by the scope of the following claims. **In** particular regard to the various functions performed by the above described features (e.g., elements, resources, etc.), the terms used to describe such features are intended to correspond, unless otherwise indicated, to any features which performs the specified function of the described features (e.g., that is functionally equivalent), even though not structurally equivalent to the disclosed structure. **In** addition, while a particular feature of the disclosure may have been disclosed with respect to only one of several implementations, such feature may be combined with one or more other features of the other implementations as may be desired and advantageous for any given or particular application.

Examples or embodiments of the subject matter and the functional operations described herein can be implemented in digital electronic circuitry, or in computer software, firmware, or hardware, including the structures disclosed in this specification and their structural equivalents, or in combinations of one or more of them.

Some examples or embodiments are implemented using one or more modules of computer program instructions encoded on a computer-readable medium for execution by, or to control the operation of, a data processing apparatus. The computer-readable medium can be a manufactured product, such as hard drive in a computer system or an embedded system. The computer-readable medium can be acquired separately and later encoded with the one or more modules of computer program instructions, such as by delivery of the one or more modules of computer program instructions over a wired or wireless network. The computer-readable medium can be a machine-readable storage device, a machine-readable storage substrate, a memory device, or a combination of one or more of them.

The terms "computing device" and "data processing apparatus" encompass all apparatus, devices, and machines for processing data, including by way of example a programmable processor, a computer, or multiple processors or computers. The apparatus can include, in addition to hardware, code that creates an execution environment for the computer program in question, e.g., code that constitutes processor firmware, a protocol stack, a database management system, an operating system, a runtime environment, or a combination of one or more of them. In addition, the apparatus can employ various different computing model infrastructures, such as web services, distributed computing and grid computing infrastructures.

The processes and logic flows described in this specification can be performed by one or more programmable processors executing one or more computer programs to perform functions by operating on input data and generating output.

Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for performing instructions and one or more memory devices for storing instructions and data. Generally, a computer will also include, or be operatively coupled to receive data from or transfer data to, or both, one or more mass storage devices for storing data, e.g., magnetic, magneto-optical disks, or optical disks. However, a computer need not have such devices. Devices suitable for storing computer program instructions and data include all forms of non-volatile memory, media and memory devices.

In the present specification "comprise" means "includes or consists of" and "comprising" means "including or consisting of".

## Claims

1. A cell lysis system comprising:
a driver apparatus (2) which incorporates:
a plurality of driver output terminals (36, 37) which provide an electrical connection between the driver apparatus (2) and a cell lysis device (3) to drive an ultrasonic transducer (12) within the cell lysis device (3);
an AC driver (38) which generates an AC drive signal at a predetermined frequency and outputs the AC drive signal at the driver output terminals (36, 37) to drive the ultrasonic transducer (12) within the cell lysis device (3);
an active power monitoring arrangement (39) which monitors the active power used by the ultrasonic transducer (12) when the ultrasonic transducer (12) is driven by the AC drive signal, wherein the active power monitoring arrangement (39) provides a monitoring signal which is indicative of an active power used by the ultrasonic transducer (12);
a processor (40) which controls the AC driver (38) and receives the monitoring signal from the active power monitoring arrangement (39); and
a memory (41) storing instructions which, when executed by the processor (40), cause the processor (40) to:
A. control the AC driver (38) to output an AC drive signal to the ultrasonic transducer (12) at a predetermined sweep frequency;
B. calculate the active power being used by the ultrasonic transducer (12) based on the monitoring signal;
C. control the AC driver (38) to modulate the AC drive signal to maximise the active power being used by the ultrasonic transducer (12);
D. store a record in the memory (41) of the maximum active power used by the ultrasonic transducer (12) and the sweep frequency of the AC drive signal;
E. repeat steps A-D for a predetermined number of iterations with the sweep frequency incrementing with each iteration such that, after the predetermined number of iterations has occurred, the sweep frequency has been incremented from a start sweep frequency to an end sweep frequency;
F. identify from the records stored in the memory (41) the optimum frequency for the AC drive signal which is the sweep frequency of the AC drive signal at which a maximum active power is used by the ultrasonic transducer (12); and
G. control the AC driver (38) to output an AC drive signal to the ultrasonic transducer (12) at the optimum frequency.

2. The system of claim 1, wherein the active power monitoring arrangement (39) comprises:
a current sensing arrangement which senses a drive current of the AC drive signal driving the ultrasonic transducer (12), wherein the active power monitoring arrangement (39) provides a monitoring signal which is indicative of the sensed drive current.

3. The system of claim 1 or claim 2, wherein the memory (41) stores instructions which, when executed by the processor (40), cause the processor (40) to:
repeat steps A-D with the sweep frequency being incremented from a start sweep frequency of 2800kHz to an end sweep frequency of 3200kHz.

4. The system of any one of the preceding claims, wherein the memory (41) stores instructions which, when executed by the processor (40), cause the processor (40) to:
in step G, control the AC driver (38) to output an AC drive signal to the ultrasonic transducer (12) at frequency which is shifted by between 1-10% of the optimum frequency.

5. The system of any one of the preceding claims, wherein the AC driver (38) modulates the AC drive signal by pulse width modulation to maximise the active power being used by the ultrasonic transducer (12).

6. The system of any one of the preceding claims, wherein the memory (41) stores instructions which, when executed by the processor (40), cause the processor (40) to:
control the AC driver (38) to alternately output an AC drive signal to the ultrasonic transducer (12) at the optimum frequency for a first predetermined length of time and to not output an AC drive signal to the ultrasonic transducer (12) for a second predetermined length of time.

7. The system of claim 6, wherein the memory (41) stores instructions which, when executed by the processor (40), cause the processor (40) to:
alternately output the AC drive signal and to not output the AC drive signal according to an operating mode selected from:
| Operating mode | First predetermined length of time (seconds) | Second predetermined length of time (seconds) |
|---|---|---|
| 1 | 4 | 2 |
| 2 | 3 | 2 |
| 3 | 2 | 2 |
| 4 | 1 | 2 |
| 5 | 1 | 1 |
| 6 | 2 | 1 |
| 7 | 3 | 1 |
| 8 | 4 | 1 |
| 9 | 4 | 3 |
| 10 | 3 | 3 |
| 11 | 2 | 3 |
| 12 | 1 | 3 |

8. The system of any one of the preceding claims, wherein the system further comprises:
a cell lysis device (3) which is releasably attached to the driver apparatus (2), the cell lysis device (3) comprising:
a housing (7);
a plurality of electrical terminals (16, 17) which are connected electrically to the plurality of driver output terminals (36, 37);
a sonication chamber (11) provided within the housing (7), the sonication chamber (11) being at least partly filled with an ultrasonic wave transfer medium, wherein the housing (7) comprises an opening (20) which is configured to receive a sample container (22) such that a part of the sample container (22) projects into the ultrasonic wave transfer medium;
an ultrasonic transducer (12) which generates ultrasonic waves in the ultrasonic wave transfer medium within the sonication chamber, wherein the ultrasonic waves are transferred by the ultrasonic wave transfer medium from the ultrasonic transducer (12) to the sample container (22) to lyse cells when cells are contained within the sample container (22).

9. The system of claim 8, wherein the driver apparatus (2) comprises a first interference fit attachment (4) and the cell lysis device (3) comprises a second interference fit attachment (5), and wherein the first interference fit attachment (4) releasably attaches to the second interference fit attachment (5) to releasably attach the cell lysis device (3) to the driver apparatus (2).

10. A method of lysing cells in a sample, the method comprising:
placing a liquid sample containing cells to be lysed in a sample container (22);
positioning the sample container (22) through an opening (20) in a housing (7) of a cell lysis device (3) such that a part of the sample container (22) projects into an ultrasonic wave transfer medium provided in a sonication chamber (11) within the housing (7); and
attaching the cell lysis device (3) to the driver apparatus (2) of any one of the preceding claims, the driver apparatus (2) incorporating:
an AC driver (38) which generates an AC drive signal at a predetermined frequency and outputs the AC drive signal at the driver output terminals (36, 37) to drive an ultrasonic transducer (12) within the cell lysis device (3);
an active power monitoring arrangement (39) which monitors the active power used by the ultrasonic transducer (12) when the ultrasonic transducer (12) is driven by the AC drive signal, wherein the active power monitoring arrangement (39) provides a monitoring signal which is indicative of an active power used by the ultrasonic transducer (12), wherein the method further comprises:
A. controlling, by a processor (40), the AC driver (38) to output an AC drive signal to the ultrasonic transducer (12) at a predetermined sweep frequency;
B. calculating, by the processor (40), the active power being used by the ultrasonic transducer (12) based on the monitoring signal;
C. controlling, by the processor (40), the AC driver (38) to modulate the AC drive signal to maximise the active power being used by the ultrasonic transducer (12);
D. storing a record in a memory (41) of the maximum active power used by the ultrasonic transducer (12) and the sweep frequency of the AC drive signal;
E. repeating steps A-D for a predetermined number of iterations with the sweep frequency incrementing with each iteration such that, after the predetermined number of iterations has occurred, the sweep frequency has been incremented from a start sweep frequency to an end sweep frequency;
F. identifying, by the processor (40), from the records stored in the memory (41) the optimum frequency for the AC drive signal which is the sweep frequency of the AC drive signal at which a maximum active power is used by the ultrasonic transducer (12); and
G. controlling, by the processor (40), the AC driver (38) to output an AC drive signal to the ultrasonic transducer (12) at the optimum frequency.

11. The method of claim 10, wherein the method further comprises:
repeating steps A-D with the sweep frequency being incremented from a start sweep frequency of 2800kHz to an end sweep frequency of 3200kHz.

12. The method of claim 10 or claim 11, wherein the method further comprises:
controlling, by the processor (40), the AC driver (38) to alternately output an AC drive signal to the ultrasonic transducer (12) at the optimum frequency for a first predetermined length of time and to not output an AC drive signal to the ultrasonic transducer (12) for a second predetermined length of time.

## Patentansprüche

1. Zellsystem zur Lyse, umfassend:
eine Treibereinrichtung (2), die enthält:
eine Vielzahl von Treiberausgangsanschlüssen (36, 37), die eine elektrische Verbindung zwischen der Treibereinrichtung (2) und einer Zelllysevorrichtung (3) bereitstellen, um einen Ultraschallwandler (12) innerhalb der Zelllysevorrichtung (3) anzutreiben;
einen AC-Treiber (38), der ein AC-Antriebssignal mit einer vorbestimmten Frequenz erzeugt und das AC-Antriebssignal an den Treiberausgangsanschlüssen (36, 37) ausgibt, um den Ultraschallwandler (12) innerhalb der Zelllysevorrichtung (3) anzutreiben;
eine Wirkleistungsüberwachungsanordnung (39), die die von dem Ultraschallwandler (12) verwendete Wirkleistung überwacht, wenn der Ultraschallwandler (12) durch das AC-Antriebssignal angetrieben wird, wobei die Wirkleistungsüberwachungsanordnung (39) ein Überwachungssignal bereitstellt, das eine von dem Ultraschallwandler (12) verwendete Wirkleistung anzeigt;
einen Prozessor (40), der den AC-Treiber (38) steuert und das Überwachungssignal von der Wirkleistungsüberwachungsanordnung (39) empfängt und
einen Speicher (41), der Anweisungen speichert, die, wenn sie durch den Prozessor (40) ausgeführt werden, den Prozessor (40) veranlassen zum:
A. Steuern des AC-Treibers (38), um ein AC-Antriebssignal mit einer zuvor bestimmten Wobbelfrequenz an den Ultraschallwandler (12) auszugeben;
B. Berechnen der Wirkleistung, die durch den Ultraschallwandler (12) verwendet wird, basierend auf dem Überwachungssignal;
C. Steuern des AC-Treibers (38), um das AC-Antriebssignal zu modulieren, um die Wirkleistung zu maximieren, die durch den Ultraschallwandler (12) verwendet wird;
D. Speichern eines Datensatzes der maximalen Wirkleistung, die durch den Ultraschallwandler (12) verwendet wird, und der Wobbelfrequenz des AC-Antriebssignals in dem Speicher (41);
E. Wiederholen der Schritte A-D für eine zuvor bestimmte Anzahl von Iterationen, wobei die Wobbelfrequenz mit jeder Iteration derart erhöht wird, dass nach Ablauf der zuvor bestimmten Anzahl von Iterationen die Wobbelfrequenz von einer Startwobbelfrequenz auf eine Endwobbelfrequenz erhöht wurde;
F. Identifizieren, aus den Datensätzen, die in dem Speicher (41) gespeichert sind, der optimalen Frequenz für das AC-Antriebssignal, die die Wobbelfrequenz des AC-Antriebssignal ist, bei der eine maximale aktive Leistung durch den Ultraschallwandler (12) verwendet wird; und
G. Steuern des AC-Treibers (38) zur Ausgabe eines AC-Antriebssignals an den Ultraschallwandler (12) mit der optimalen Frequenz.

2. System nach Anspruch 1, wobei die Wirkleistungsüberwachungsanordnung (39) umfasst:
eine Stromerfassungsanordnung, einen Antriebsstrom des AC-Antriebssignals erfasst, das den Ultraschallwandler (12) antreibt, wobei die Wirkleistungsüberwachungsanordnung (39) ein Überwachungssignal bereitstellt, das den erfassten Antriebsstrom anzeigt.

3. System nach Anspruch 1 oder 2, wobei der Speicher (41) Anweisungen speichert, die, wenn sie durch den Prozessor (40) ausgeführt werden, den Prozessor (40) veranlassen zum:
Wiederholen der Schritte A-D, wobei die Wobbelfrequenz von einer Startwobbelfrequenz von 2800 kHz auf eine Endwobbelfrequenz von 3200 kHz inkrementiert wird.

4. System nach einem der vorstehenden Ansprüche, wobei der Speicher (41) Anweisungen speichert, die, wenn sie durch den Prozessor (40) ausgeführt werden, den Prozessor (40) veranlassen zum:
in Schritt G, Steuern des AC-Treibers (38) zur Ausgabe eines AC-Antriebssignals an den Ultraschallwandler (12) mit einer Frequenz, die um zwischen 1 und 10 % der optimalen Frequenz verschoben ist

5. System nach einem der vorstehenden Ansprüche, wobei der AC-Treiber (38) das AC-Treibersignal durch Pulsweitenmodulation moduliert, um die von dem Ultraschallwandler (12) verwendete Wirkleistung zu maximieren.

6. System nach einem der vorstehenden Ansprüche, wobei der Speicher (41) Anweisungen speichert, die, wenn sie durch den Prozessor (40) ausgeführt werden, den Prozessor (40) veranlassen zum:
Steuern des AC-Treibers (38), um abwechselnd für eine erste vorbestimmte Zeitdauer ein AC-Antriebssignal mit der optimalen Frequenz an den Ultraschallwandler (12) auszugeben und für eine zweite vorbestimmte Zeitdauer kein AC-Antriebssignal an den Ultraschallwandler (12) auszugeben.

7. System nach Anspruch 6, wobei der Speicher (41) Anweisungen speichert, die, wenn sie durch den Prozessor (40) ausgeführt werden, den Prozessor (40) veranlassen zum:
abwechselnden Ausgeben des AC-Treibersignals und Nicht-Ausgaben des AC-Treibersignals gemäß einem Betriebsmodus, ausgewählt aus:
| Betriebsmodus | Erste vorbestimmte Zeitdauer (Sekunden) | Zweite vorbestimmte Zeitdauer (Sekunden) |
|---|---|---|
| 1 | 4 | 2 |
| 2 | 3 | 2 |
| 3 | 2 | 2 |
| 4 | 1 | 2 |
| 5 | 1 | 1 |
| 6 | 2 | 1 |
| 7 | 3 | 1 |
| 8 | 4 | 1 |
| 9 | 4 | 3 |
| 10 | 3 | 3 |
| 11 | 2 | 3 |
| 12 | 1 | 3 |

8. System nach einem der vorstehenden Ansprüche, wobei das System ferner umfasst:
eine Zelllysevorrichtung (3), die lösbar an der Treibereinrichtung (2) angebracht ist, wobei die Zelllysevorrichtung (3) umfasst:
ein Gehäuse (7);
eine Vielzahl von elektrischen Anschlüssen (16, 17), die mit der Vielzahl von Treiberausgangsanschlüssen (36, 37) elektrisch verbunden sind;
eine Beschallungskammer (11), bereitgestellt innerhalb des Gehäuses (7), wobei die Beschallungskammer (11) mindestens teilweise mit einem Ultraschallwellenübertragungsmedium gefüllt ist, wobei das Gehäuse (7) eine Öffnung (20) umfasst, die konfiguriert ist, um einen Probenbehälter (22) aufzunehmen, sodass ein Teil des Probenbehälters (22) in das Ultraschallwellenübertragungsmedium hineinragt;
ein Ultraschallwandler (12), der Ultraschallwellen in dem Ultraschallwellenübertragungsmedium innerhalb der Beschallungskammer erzeugt, wobei die Ultraschallwellen durch das Ultraschallwellenübertragungsmedium von dem Ultraschallwandler (12) zu dem Probenbehälter (22) übertragen werden, um Zellen zu lysieren, wenn Zellen innerhalb des Probenbehälters (22) enthalten sind.

9. System nach Anspruch 8, wobei die Treibereinrichtung (2) eine erste Presspassungsbefestigung (4) umfasst und die Zelllysevorrichtung (3) eine zweite Presspassungsbefestigung (5) umfasst, und wobei die erste Presspassungsbefestigung (4) lösbar an der zweiten Presspassungsbefestigung (5) befestigt wird, um die Zelllysevorrichtung (3) lösbar an der Treibereinrichtung (2) zu befestigen.

10. Verfahren zum Lysieren von Zellen von einer Probe, das Verfahren umfassend:
Einbringen einer flüssigen Probe, die zu lysierende Zellen enthält, in einen Probenbehälter (22);
Positionieren des Probenbehälters (22) durch eine Öffnung (20) in einem Gehäuse (7) einer Zelllysevorrichtung (3) derart, dass ein Teil des Probenbehälters (22) in ein in einer Beschallungskammer (11) innerhalb des Gehäuses (7) bereitgestelltes Ultraschallwellenübertragungsmedium hineinragt; und
Befestigen der Zelllysevorrichtung (3) an der Treibereinrichtung (2) nach einem der vorstehenden Ansprüche, wobei die Treibereinrichtung (2) enthält:
einen AC-Treiber (38), der ein AC-Antriebssignal mit einer vorbestimmten Frequenz erzeugt und das AC-Antriebssignal an den Treiberausgangsanschlüssen (36, 37) ausgibt, um einen Ultraschallwandler (12) innerhalb der Zelllysevorrichtung (3) anzutreiben;
eine Wirkleistungsüberwachungsanordnung (39), die die von dem Ultraschallwandler (12) verwendete Wirkleistung überwacht, wenn der Ultraschallwandler (12) durch das AC-Antriebssignal angetrieben wird, wobei die Wirkleistungsüberwachungsanordnung (39) ein Überwachungssignal bereitstellt, das eine von dem Ultraschallwandler (12) verwendete Wirkleistung anzeigt, wobei das Verfahren ferner umfasst:
A. Steuern durch einen Prozessor (40) des AC-Treibers (38), um ein AC-Antriebssignal mit einer zuvor bestimmten Wobbelfrequenz an den Ultraschallwandler (12) auszugeben;
B. Berechnen durch den Prozessor (40) der Wirkleistung, die durch den Ultraschallwandler (12) verwendet wird, basierend auf dem Überwachungssignal;
C. Steuern durch den Prozessor (40) des AC-Treibers (38), um das AC-Antriebssignal zu modulieren, um die Wirkleistung zu maximieren, die durch den Ultraschallwandler (12) verwendet wird;
D. Speichern eines Datensatzes der maximalen Wirkleistung, die durch den Ultraschallwandler (12) verwendet wird, und der Wobbelfrequenz des AC-Antriebssignals in einem Speicher (41);
E. Wiederholen der Schritte A-D für eine zuvor bestimmte Anzahl von Iterationen, wobei die Wobbelfrequenz mit jeder Iteration derart erhöht wird, dass nach Ablauf der zuvor bestimmten Anzahl von Iterationen die Wobbelfrequenz von einer Startwobbelfrequenz auf eine Endwobbelfrequenz erhöht wurde;
F. Identifizieren durch den Prozessor (40), aus den Datensätzen, die in dem Speicher (41) gespeichert sind, der optimalen Frequenz für das AC-Antriebssignal, die die Wobbelfrequenz des AC-Antriebssignals ist, bei der eine maximale Wirkleistung durch den Ultraschallwandler (12) verwendet wird; und
G. Steuern durch den Prozessor (40) des AC-Treibers (38) zur Ausgabe eines AC-Antriebssignals an den Ultraschallwandler (12) mit der optimalen Frequenz.

11. Verfahren nach Anspruch 10, wobei das Verfahren ferner umfasst:
Wiederholen der Schritte A-D, wobei die Wobbelfrequenz von einer Startwobbelfrequenz von 2800 kHz auf eine Endwobbelfrequenz von 3200 kHz inkrementiert wird.

12. Verfahren nach Anspruch 10 oder Anspruch 11, wobei das Verfahren ferner umfasst:
Steuern durch den Prozessor (40) des AC-Treibers (38), um abwechselnd für eine erste vorbestimmte Zeitdauer ein AC-Antriebssignal mit der optimalen Frequenz an den Ultraschallwandler (12) auszugeben und für eine zweite vorbestimmte Zeitdauer kein AC-Antriebssignal an den Ultraschallwandler (12) auszugeben.

## Revendications

1. Système de lyse cellulaire comprenant :
un appareil d'entraînement (2) qui incorpore :
une pluralité de bornes de sortie d'entraînement (36, 37) qui fournissent une connexion électrique entre l'appareil d'entraînement (2) et un dispositif de lyse cellulaire (3) pour entraîner un transducteur à ultrasons (12) au sein du dispositif de lyse cellulaire (3) ;
un dispositif d'entraînement CA (38) qui génère un signal d'entraînement CA à une fréquence prédéterminée et délivre le signal d'entraînement CA aux bornes de sortie d'entraînement (36, 37) pour entraîner le transducteur à ultrasons (12) au sein du dispositif de lyse cellulaire (3) ;
un agencement de surveillance de puissance active (39) qui surveille la puissance active utilisée par le transducteur à ultrasons (12) lorsque le transducteur à ultrasons (12) est entraîné par le signal d'entraînement CA, dans lequel l'agencement de surveillance de puissance active (39) fournit un signal de surveillance qui indique une puissance active utilisée par le transducteur à ultrasons (12) ;
un processeur (40) qui commande le dispositif d'entraînement CA (38) et reçoit le signal de surveillance de l'agencement de surveillance de puissance active (39) ; et
une mémoire (41) stockant des instructions qui, lorsqu'elles sont exécutées par le processeur (40), amènent le processeur (40) à :
A. commander le dispositif d'entraînement CA (38) afin de délivrer un signal d'entraînement CA au transducteur à ultrasons (12) à une fréquence de balayage prédéterminée ;
B. calculer la puissance active qui est utilisée par le transducteur à ultrasons (12) en fonction du signal de surveillance ;
C. commander le dispositif d'entraînement CA (38) afin de moduler le signal d'entraînement CA pour maximiser la puissance active qui est utilisée par le transducteur à ultrasons (12) ;
D. stocker un enregistrement dans la mémoire (14) de la puissance active maximale utilisée par le transducteur à ultrasons (12) et de la fréquence de balayage du signal d'entraînement CA ;
E. répéter les étapes A à D un nombre prédéterminé d'itérations, la fréquence de balayage augmentant par incrément avec chaque itération de telle sorte que, après que le nombre prédéterminé d'itérations s'est produit, la fréquence de balayage a été incrémentée d'une fréquence de balayage de départ à une fréquence de balayage de fin ;
F. identifier à partir des enregistrements stockés dans la mémoire (41) la fréquence optimale pour le signal d'entraînement CA, qui est la fréquence de balayage du signal d'entraînement CA à laquelle une puissance active maximale est utilisée par le transducteur à ultrasons (12) ; et
G. commander le dispositif d'entraînement CA (38) afin de délivrer un signal d'entraînement CA au transducteur à ultrasons (12) à la fréquence optimale.

2. Système selon la revendication 1, dans lequel l'agencement de surveillance de puissance active (39) comprend :
un agencement de détection de courant qui détecte un courant d'entraînement du signal d'entraînement CA entraînant le transducteur à ultrasons (12), dans lequel l'agencement de surveillance de puissance active (39) fournit un signal de surveillance qui indique le courant d'entraînement détecté.

3. Système selon la revendication 1 ou la revendication 2, dans lequel la mémoire (41) stocke des instructions qui, lorsqu'elles sont exécutées par le processeur (40), amènent le processeur (40) à :
répéter les étapes A à D, la fréquence de balayage augmentant par incrément d'une fréquence de balayage de départ de 2 800 kHz à une fréquence de balayage de fin de 3 200 kHz.

4. Système selon l'une quelconque des revendications précédentes, dans lequel la mémoire (41) stocke des instructions qui, lorsqu'elles sont exécutées par le processeur (40), amènent le processeur (40) à :
à l'étape G, commander le circuit d'entraînement CA (38) pour délivrer un signal d'entraînement CA au transducteur à ultrasons (12) à une fréquence qui est décalée de 1 à 10 % de la fréquence optimale.

5. Système selon l'une quelconque des revendications précédentes, dans lequel le dispositif d'entraînement CA (38) module le signal d'entraînement CA par modulation de largeur d'impulsion pour maximiser la puissance active qui est utilisée par le transducteur à ultrasons (12).

6. Système selon l'une quelconque des revendications précédentes, dans lequel la mémoire (41) stocke des instructions qui, lorsqu'elles sont exécutées par le processeur (40), amènent le processeur (40) à :
commander le circuit d'entraînement CA (38) pour, en alternance, délivrer un signal d'entraînement CA au transducteur à ultrasons (12) à la fréquence optimale pendant une première durée prédéterminée et ne pas délivrer de signal d'entraînement CA au transducteur à ultrasons (12) pendant une seconde durée prédéterminée.

7. Système selon la revendication 6, dans lequel la mémoire (41) stocke des instructions qui, lorsqu'elles sont exécutées par le processeur (40), amènent le processeur (40) à :
en alternance, délivrer le signal d'entraînement CA et ne pas délivrer le signal d'entraînement CA selon un mode de fonctionnement choisi parmi :
| Mode de fonctionnement | Première durée prédéterminée (secondes) | Seconde durée prédéterminée (secondes) |
|---|---|---|
| 1 | 4 | 2 |
| 2 | 3 | 2 |
| 3 | 2 | 2 |
| 4 | 1 | 2 |
| 5 | 1 | 1 |
| 6 | 2 | 1 |
| 7 | 3 | 1 |
| 8 | 4 | 1 |
| 9 | 4 | 3 |
| 10 | 3 | 3 |
| 11 | 2 | 3 |
| 12 | 1 | 3 |

8. Système selon l'une quelconque des revendications précédentes, dans lequel le système comprend en outre :
un dispositif de lyse cellulaire (3) qui est fixé de manière amovible à l'appareil d'entraînement (2), le dispositif de lyse cellulaire (3) comprenant :
un boîtier (7) ;
une pluralité de bornes électriques (16, 17) qui sont connectées électriquement à la pluralité de bornes de sortie d'entraînement (36, 37) ;
une chambre de sonication (11) disposée au sein du boîtier (7), la chambre de sonication (11) étant au moins partiellement remplie d'un milieu de transfert d'ondes ultrasoniques, dans lequel le boîtier (7) comprend une ouverture (20) qui est conçue pour recevoir un récipient à échantillon (22) de telle sorte qu'une partie du récipient à échantillon (22) fait saillie dans le milieu de transfert d'ondes ultrasoniques ;
un transducteur à ultrasons (12) qui génère des ondes ultrasoniques dans le milieu de transfert d'ondes ultrasoniques au sein de la chambre de sonication, dans lequel les ondes ultrasoniques sont transférées par le milieu de transfert d'ondes ultrasoniques, du transducteur à ultrasons (12) au récipient à échantillons (22), pour lyser des cellules lorsque des cellules sont contenues au sein du récipient à échantillon (22).

9. Système selon la revendication 8, dans lequel l'appareil d'entraînement (2) comprend un premier accessoire d'ajustement serré (4) et le dispositif de lyse cellulaire (3) comprend un second accessoire d'ajustement serré (5), et dans lequel le premier accessoire d'ajustement serré (4) se fixe de manière amovible au second ajustement serré l'accessoire (5) pour fixer de manière amovible le dispositif de lyse cellulaire (3) à l'appareil d'entraînement (2).

10. Procédé de lyse de cellules dans un échantillon, le procédé comprenant :
le placement d'un échantillon liquide contenant des cellules à lyser dans un récipient à échantillon (22) ;
le positionnement du récipient à échantillon (22) à travers une ouverture (20) dans un boîtier (7) d'un dispositif de lyse cellulaire (3) de telle sorte qu'une partie du récipient à échantillon (22) fait saillie dans un milieu de transfert d'ondes ultrasoniques disposé dans une chambre de sonication (11) au sein du boîtier (7) ; et
la fixation du dispositif de lyse cellulaire (3) à l'appareil d'entraînement (2) selon l'une quelconque des revendications précédentes, l'appareil d'entraînement (2) incorporant :
un dispositif d'entraînement CA (38) qui génère un signal d'entraînement CA à une fréquence prédéterminée et délivre le signal d'entraînement CA aux bornes de sortie d'entraînement (36, 37) pour entraîner un transducteur à ultrasons (12) au sein du dispositif de lyse cellulaire (3) ;
un agencement de surveillance de puissance active (39) qui surveille la puissance active utilisée par le transducteur à ultrasons (12) lorsque le transducteur à ultrasons (12) est entraîné par le signal d'entraînement CA, dans lequel l'agencement de surveillance de puissance active (39) fournit un signal de surveillance qui indique une puissance active utilisée par le transducteur à ultrasons (12), dans lequel le procédé comprend en outre :
A. la commande, par un processeur (40), du dispositif d'entraînement CA (38), pour délivrer un signal d'entraînement CA au transducteur à ultrasons (12) à une fréquence de balayage prédéterminée ;
B. le calcul, par le processeur (40), de la puissance active qui est utilisée par le transducteur à ultrasons (12) en fonction du signal de surveillance ;
C. la commande, par le processeur (40), du dispositif d'entraînement CA (38) pour moduler le signal d'entraînement CA afin de maximiser la puissance active qui est utilisée par le transducteur à ultrasons (12) ;
D. le stockage d'un enregistrement dans une mémoire (41) de la puissance active maximale utilisée par le transducteur à ultrasons (12) et de la fréquence de balayage du signal d'entraînement CA ;
E. la répétition des étapes A à D un nombre prédéterminé d'itérations, la fréquence de balayage augmentant par incrément avec chaque itération de telle sorte que, après que le nombre prédéterminé d'itérations s'est produit, la fréquence de balayage a été incrémentée d'une fréquence de balayage de départ à une fréquence de balayage de fin ;
F. l'identification, par le processeur (40), à partir des enregistrements stockés dans la mémoire (41), de la fréquence optimale pour le signal d'entraînement CA, qui est la fréquence de balayage du signal d'entraînement CA à laquelle une puissance active maximale est utilisée par le transducteur à ultrasons (12) ; et
G. la commande, par le processeur (40), du dispositif d'entraînement CA (38) pour délivrer un signal d'entraînement CA au transducteur à ultrasons (12) à la fréquence optimale.

11. Procédé selon la revendication 10, dans lequel le procédé comprend en outre :
la répétition des étapes A à D, la fréquence de balayage augmentant par incrément d'une fréquence de balayage de départ de 2 800 kHz à une fréquence de balayage de fin de 3 200 kHz.

12. Procédé selon la revendication 10 ou la revendication 11, dans lequel le procédé comprend en outre :
la commande, par le processeur (40), du circuit d'entraînement CA (38) pour, en alternance, délivrer un signal d'entraînement CA au transducteur à ultrasons (12) à la fréquence optimale pendant une première durée prédéterminée et ne pas délivrer de signal d'entraînement CA au transducteur à ultrasons (12) pendant une seconde durée prédéterminée.
